# FASCICULE DE BREVET EUROPEEN

(11) **EP 1 216 983 B1**
(45) Date de publication et mention de la délivrance du brevet: **01.06.2005**
(21) Numéro de dépôt: 01403256.9
(22) Date de dépôt: 14.12.2001
(51) Int. Cl.: C07C 68/02, C07C 69/96

(54) **Procédé de synthèse de chloroformiates aliphatiques, cycloaliphatiques ou araliphatiques**
Verfahren zur Herstellung von aliphatischen, zykloaliphatischen oder araliphatischen Chlorameisensäureestern
Process for the preparation of aliphatic, cycloaliphatic or araliphatic chloroformates

(30) Priorité: 22.12.2000 FR 0016880
(43) Date de publication de la demande: 26.06.2002
(73) Titulaire: SNPE, 75181 Paris Cedex 04 (FR)
(72) Inventeur: Bonnard, Hubert, 91590 La Ferte Alais (FR); Ferruccio, Laurence, 91810 Vert Le Grand (FR); Gauthier, Patricia, Cerny, 91590 La Ferte Alais (FR); Senet, Jean-Pierre, 77760 Chapelle La Reine (FR)
(74) Mandataire: Ahner, Francis

(56) Documents cités:
- EP-A- 0 075 145
- FR-A- 2 484 406

## Description

La présente invention concerne un procédé de synthèse de chloroformiates aliphatiques, cycloaliphatiques ou araliphatiques, substitués ou non.

Plus particulièrement, elle concerne un procédé de synthèse améliorée de ces chloroformiates par phosgénation des alcools correspondants.

Les chloroformiates sont des intermédiaires de synthèse très utiles dans l'industrie pharmaceutique, cosmétique et alimentaire.

On citera notamment le chloroformiate de benzyle, une des matières premières pour la synthèse de l'acide Z-aspartique, composé qui entre dans la fabrication de l'aspartame.

Un autre chloroformiate très important est le chloroformiate de menthyle, composé utilisé pour son goût mentholé. On le retrouve comme intermédiaire de synthèse dans l'industrie du tabac et dans l'industrie pharmaceutique.

L'homme du métier connaît déjà des procédés de synthèse de chloroformiates, à partir de phosgène et de l'alcool correspondant. Le problème rencontré lors de telles synthèses est la production d'acide chlorhydrique qui conduit à la formation de produits secondaires.

Une première solution à ce problème est d'ajouter une base, comme une amine tertiaire, au milieu réactionnel. Il est ainsi décrit dans un article du Journal of Organic Chemistry de 1993 (vol.58 n° 8, 2186 - 2195) une synthèse de chloroformiate de menthyle : la réaction du menthol et du phosgène est effectuée dans du toluène, en présence de quinoléine.

Un autre article du Journal of Organic Chemistry de 1998 (vol. 63, 3235 - 3250) décrit une synthèse du chloroformiate de menthyle à partir de phosgène et de menthol faisant également intervenir une base, la pyridine. Les inconvénients liés à de telles synthèses sont non seulement le coût supplémentaire dû à l'utilisation d'une base, mais aussi l'obligation d'éliminer le chlorhydrate de l'amine formé, notamment par filtration directe ou par lavage à l'eau de la phase organique. Il en résulte un procédé plus long et plus onéreux.

D'autres voies de synthèse ne faisant pas intervenir de base ont aussi été proposées. Ainsi, le brevet US 3 419 543 divulgue un procédé de synthèse du chloroformiate de menthyle, sans base, à partir de phosgène et d'une solution de menthol dans du cyclopentane. Dans la réaction ainsi décrite, les constituants sont introduits à - 75°C. Cette très basse température est un inconvénient car elle est difficile à obtenir industriellement, cela nécessitant un équipement particulier et très coûteux.

Une autre voie de synthèse a alors été proposée, à une température supérieure et ne faisant pas non plus intervenir de base. Un article paru dans le volume 54 du Tetrahedron en 1998 (Tetrahedron 54 (1998) 10537-10534) divulgue une telle synthèse : le phosgène réagit à - 10°C avec le menthol, dans du toluène. On obtient du chloroformiate de menthyle avec un rendement de 82 % seulement.

De plus, ces procédés, à pression atmosphérique et sans base, présentent une cinétique de réaction très lente, ce qui est inconvénient au niveau industriel.

Selon le procédé de la demande de brevet FR 2 484 406, les chloroformiates facilement décomposables sont obtenus en introduisant simultanément l'alcool et le phosgène dans une phase liquide de chloroformiate à une température de 20° à 50°C et sous une pression de préférence comprise entre 0,9 et 2 atmosphères. Ce procédé est difficile à mettre en oeuvre. La vitesse d'alimentation doit se situer dans une gamme précise et par conséquent la vitesse de réaction doit être contrôlée en permanence. Un temps de réaction très long, de l'ordre de 16 heures est nécessaire.

De même, le procédé décrit dans la demande de brevet EP 75145 nécessite des installations particulières pour introduire l'alcool et le phosgène à contre-courant et pour contrôler la teneur en phosgène qui doit toujours être comprise entre 3 et 20 % en poids dans le mélange réactionnel. Le procédé est de plus limité à la production des chloroformiates d'alkyle.

L'homme du métier est donc toujours à la recherche d'un procédé de synthèse de chloroformiates aliphatiques, cycloaliphatiques ou araliphatiques, peu coûteux, avec une cinétique rapide, un haut rendement et donnant des produits de très grande pureté.

La présente invention a pour objet un tel procédé.

L'invention concerne un procédé de synthèse de chloroformiates aliphatiques, cycloaliphatiques ou araliphatiques, substitués ou non, par réaction de l'alcool correspondant avec du phosgène, du diphosgène et/ou du triphosgène, caractérisé en ce que la réaction est effectuée sous une pression comprise entre 35.10³Pa et 75.10³Pa, soit entre 350 mbar et 750 mbar et à une température comprise entre - 10°C et + 20°C.

Ce procédé présente l'avantage d'être simple et peu coûteux. Les chloroformiates sont obtenus, rapidement, avec un rendement très élevé, généralement supérieur à 97 % et ont une bonne pureté, en général de l'ordre de 97 %, voire 99 %. Très peu de produits secondaires sont obtenus. Ce résultat est d'autant plus surprenant que le vide est habituellement utilisé pour éliminer le phosgène du milieu réactionnel. Un dégazage pendant la réaction devrait donc produire un déficit en phosgène. Il est connu que dans ce cas l'alcool réagit avec le chloroformiate déjà formé pour donner un carbonate. D'autre part, la vitesse de réaction étant fonction de la concentration en phosgène, la cinétique aurait dû être ralentie.

Ce procédé permet d'obtenir les chloroformiates aliphatiques, cycloaliphatiques ou araliphatiques, substitués ou non, qui peuvent être préparés par phosgénation des alcools correspondants. Il permet, en particulier d'obtenir les chloroformiates aliphatiques en C1 à C20, cycloaliphatiques en C4 à C20, araliphatiques en C₇ à C20, saturés ou insaturés ; substitués ou non, les substituants étant des groupes non réactifs par rapport au phosgène et à l'acide chlorhydrique. Ce procédé convient particulièrement bien pour l'obtention des chloroformiates aliphatiques ou cycloaliphatiques secondaires ou tertiaires, et des chloroformiates araliphatiques. De façon particulièrement préférée, ce procédé permet d'obtenir les chloroformiates de menthyle et de benzyle.

Le phosgène, le diphosgène et/ou le triphosgène peuvent être utilisés. On utilise de préférence le phosgène. La quantité de phosgène, de diphosgène et/ou de triphosgène introduite est généralement telle qu'il y ait entre 1 et 10 équivalents molaires de phosgène par rapport à l'alcool, et, de préférence, entre 1,5 et 2,5 équivalents molaires.

Le diphosgène et le triphosgène générant respectivement 2 et 3 moles de phosgène, la quantité utilisée sera comprise entre respectivement 0,5 et 5 moles, et de préférence entre 0,75 et 1,25 moles pour le diphosgène et entre 0,3 et 3,3 moles, de préférence entre 0,5 et 0,8 mole pour le triphosgène.

La réaction peut être effectuée avec ou sans solvant. Un des composés de la réaction, le chloroformiate ou le phosgène, peut être utilisé comme solvant. De préférence, elle est effectuée en présence d'un solvant inerte choisi dans le groupe constitué par les hydrocarbures aliphatiques chlorés ou non, les hydrocarbures aromatiques chlorés ou non, les esters et les éthers.

Comme hydrocarbures aliphatiques, on peut citer le chlorure de méthylène, le chloroforme, l'heptane, le pentane.

Comme hydrocarbures aromatiques, on peut citer le toluène, le xylène. On peut également utiliser des esters, comme par exemple l'acétate d'éthyle, l'acétate d'isopropyle mais aussi le carbonate de méthyle.

Des éthers peuvent aussi servir de solvant inerte, il s'agit entre autre des éthers éthyliques et diisopropyliques.

Ce procédé convient particulièrement bien pour l'obtention des chloroformiates de menthyle et de benzyle, obtenus respectivement par réaction du menthol et de l'alcool benzylique avec le phosgène.

L'introduction du phosgène dans le réacteur est effectuée, de préférence, à une température inférieure à 8°C et sous une pression comprise entre 35.10³Pa et 75.10³Pa, soit entre 350 mbar et 750 mbar.

Quand tout le phosgène est introduit, on laisse les réactifs en contact de préférence pendant une durée comprise entre 2 et 8 heures, à une température de préférence comprise entre - 10°C et 20°C.

On obtient du chloroformiate de menthyle de très grande pureté, de l'ordre de 99 % et avec un rendement supérieur à 97 %. On retrouve dans celui-ci moins de 0,5 % en poids de menthol et seulement des traces de chlorure de menthyle et de bismenthylcarbonate.

Le chloroformiate de benzyle est également obtenu avec une bonne pureté, supérieure à 97 %. Ainsi, le chlorure de benzyle n'y est présent qu'à raison de 0,5 % en poids ou moins, et le carbonate de dibenzyle est en quantité inférieure à 0,3 % en poids.

Le faible taux de ces impuretés, et notamment du chlorure de benzyle, est un grand avantage. En effet, celui-ci est un composé très réactif et est par conséquent gênant pour certaines applications du chloroformiate de benzyle, et notamment dans les synthèses d'acides aminés.

Les exemples suivants illustrent, à titre non limitatif, des variantes de mise en oeuvre de l'invention.

### EXEMPLE 1 : Synthèse du chloroformiate de menthyle sous 500 mbar et à une température comprise entre - 3°C et 20°C.

La réaction est effectuée dans un réacteur de 500 ml à double enveloppe. Ce réacteur est équipé d'un réfrigérant surmonté d'un piège à carboglace. On utilise deux colonnes reliées au montage ainsi formé, remplies de charbon, pour piéger le phosgène et l'acide chlorhydrique dégagé pendant la réaction.

On introduit dans le réacteur 14,9 g de toluène et 50,4 g de (L)- menthol, soit 0,322 mol de menthol. Le milieu est refroidi vers - 3°C - 0°C. On introduit alors progressivement, sous une pression de 500 mbar, 65 g de phosgène, soit 0,650 mol. La quantité de phosgène est donc de 2 équivalents molaires par rapport au menthol.

Après introduction de la totalité du phosgène, on réchauffe progressivement le milieu réactionnel. Cette opération est effectuée sous une pression de 500 mbar. La température passe de - 3°C à + 7°C en 4 heures puis de 7° à 19°C en 50 minutes et enfin on maintient la température à 19°C pendant 20 minutes. Le produit obtenu est ensuite dégazé à cette température.

On obtient 70 g de chloroformiate de (L)- menthyle, soit 0,320 mol, ce qui correspond à un rendement de 99 %.

Le produit obtenu est analysé par chromatographie en phase gazeuse. La pureté du chloroformiate est de 99 %, on retrouve moins de 0,5 % en poids de menthol et 250 ppm de chlorure de menthyle. Le bismenthylcarbonate n'est pas détecté.

### EXEMPLE 2 : Synthèse du chloroformiate de menthyle sous 500 mbar et à une température comprise entre 8°C et 19°C.

Le montage utilisé est le même que celui de l'exemple 1.

On introduit dans le réacteur 93,4 g de toluène et 315,8 g de menthol, soit 2 mol. On ajoute progressivement, sous une pression de 500 mbar et à une température comprise entre 8°C et 15°C, 308 g de phosgène soit 3,1 mol, soit 1,55 équivalents molaire par rapport au menthol.

En fin d'introduction, on poursuit encore la réaction pendant 6,5 heures, à une température comprise entre 12°C et 19°C et sous une pression de 500 mbar.

On obtient 435 g de chloroformiate de menthyle, soit 1,99 mol, ce qui correspond à un rendement de 99 %.

Le produit obtenu est analysé par chromatographie en phase gazeuse. La pureté du chloroformiate est de 99,7 %, on retrouve moins de 0,3 % de menthol et moins de 150 ppm de chlorure de menthyle. Le bismenthylcarbonate n'est pas détecté.

### EXEMPLE 3 : Synthèse de chloroformiate de benzyle sous 500 mbar.

Le montage utilisé est le même que celui de l'exemple 1.

On introduit dans le réacteur 217 g d'alcool, soit 1,99 mol. Le milieu est refroidi vers 0°C.

On introduit alors progressivement, sous une pression de 500 mbar et à une température comprise entre 0°C et 6°C, 250 g de phosgène, soit 2,52 mol. La quantité de phosgène utilisée est de 1,27 équivalents molaires par rapport au menthol.

La durée totale de la réaction est de 6,5 heures.

On obtient 275 g de chloroformiate de benzyle, soit 1,61 mol, ce qui correspond à un rendement de 81 %.

Le produit obtenu est analysé par chromatographie en phase gazeuse. La pureté du chloroformiate est de 98,4 %. Le produit final contient 0,4 % d'alcool benzylique, 0,51 % de chlorure de benzyle et 0,26 % de carbonate de dibenzyle.

### EXEMPLE 4 (comparatif): Synthèse du chloroformiate de benzyle sous 250 mbar

On procède de la même manière que pour l'exemple 3. On opère sous une pression de 250 mbar.

Les quantités de réactif utilisés sont les suivantes :
- 223 g d'alcool benzylique, soit 2,06 mol, et
- 275 g de phosgène, soit 2,77 mol.

En fin d'introduction du phosgène, on poursuit encore la réaction pendant 2 heures.

On obtient 263 g de chloroformiate de benzyle, soit 1,54 mol, ce qui correspond à un rendement de 75 %.

Le produit obtenu est analysé par chromatographie en phase gazeuse. La pureté du chloroformiate est de 97,1 %.

Le produit final contient 0,02 % d'alcool benzylique, 0,27 % de chlorure de benzyle et 0,29 % de carbonate de dibenzyle.

### ESSAI A PRESSION ATMOSPHERIQUE : Synthèse du chloroformiate de menthyle.

Cet essai ne fait pas partie de l'invention, il a été réalisé dans le but de montrer que le fait de travailler sous pression réduite ne correspond pas à un choix arbitraire mais correspond à une sélection nécessaire pour obtenir l'effet technique recherché.

On introduit dans le réacteur 108 g de toluène et 100 g de menthol, soit 0,63 mol de menthol. On introduit 89 g de phosgène dans le réacteur, soit 0,9 mol, soit 1,4 équivalent molaire par rapport au menthol.

L'introduction du phosgène est effectuée à la pression atmosphérique et à une température comprise entre - 8°C et + 8°C. On poursuit encore la réaction pendant un peu plus de 4 heures, à une température comprise entre - 5°C et + 5°C.

On obtient 93,4 g de chloroformiate de menthyle, soit 0,427 mol, ce qui correspond à un rendement de 68 %. La principale impureté présente dans ce produit est du menthol de départ n'ayant pas réagi, il est présent à raison de 33 % en poids.

## Revendications

1. Procédé de synthèse de chloroformiates aliphatiques, cycloaliphatiques ou araliphatiques, substitués ou non, par réaction de l'alcool correspondant avec du phosgène, du diphosgène et/ou du triphosgène, **caractérisé en ce que** la réaction est effectuée sous une pression comprise entre 35.10³Pa et 75.10³Pa et à une température comprise entre - 10°C et + 20°C.

2. Procédé selon la revendication 1, **caractérisé en ce que** la réaction est effectuée en présence d'un solvant inerte choisi dans le groupe constitué par les hydrocarbures aliphatiques chlorés ou non, les hydrocarbures aromatiques chlorés ou non, les esters et les éthers.

3. Procédé selon la revendications 1 ou 2, **caractérisé en ce que** l'on utilise le phosgène.

4. Procédé selon l'une quelconque des revendications 1 à 3, **caractérisé en ce que** la quantité de phosgène, de diphosgène et/ou de triphosgène utilisée est telle qu'il y ait entre 1 et 10 équivalents molaires de phosgène par rapport à l'alcool.

5. Procédé selon la revendication 4, **caractérisé en ce que** la quantité de phosgène, de diphosgène et/ou de triphosgène utilisée est telle qu'il y ait entre 1,5 et 2,5 équivalents molaires de phosgène par rapport à l'alcool.

6. Procédé selon l'une quelconque des revendications 1 à 5, **caractérisé en ce que** le chloroformiate obtenu est un chloroformiate aliphatique ou cycloaliphatique secondaire ou tertiaire.

7. Procédé selon l'une quelconque des revendications 1 à 6, **caractérisé en ce que** le chloroformiate obtenu est le chloroformiate de menthyle.

## Patentansprüche

1. Verfahren zur Synthese von substituierten oder unsubstituierten aliphatischen, cycloaliphatischen oder araliphatischen Chlorformiaten durch Umsetzung des entsprechenden Alkohols mit Phosgen, Diphosgen und/oder Triphosgen, **dadurch gekennzeichnet, dass** die Umsetzung unter einem Druck zwischen 35·10³ Pa und 75·10³ Pa einschließlich und bei einer Temperatur zwischen -10 °C und +20 °C einschließlich bewirkt wird.

2. Verfahren nach Anspruch 1, **dadurch gekennzeichnet, dass** die Reaktion in Anwesenheit eines inerten Lösungsmittels bewirkt wird, das aus der Gruppe ausgewählt ist, die aus chlorierten oder nicht chlorierten aliphatischen Kohlenwasserstoffen, chlorierten oder nicht chlorierten aromatischen Kohlenwasserstoffen, Estern und Ethern besteht.

3. Verfahren nach Anspruch 1 oder 2, **dadurch gekennzeichnet, dass** man Phosgen verwendet.

4. Verfahren nach irgendeinem der Ansprüche 1 bis 3, **dadurch gekennzeichnet, dass** die verwendete Menge an Phosgen, Diphosgen und/oder Triphosgen derart ist, dass sie zwischen 1 und 10 Moläquivalenten Phosgen, bezogen auf den Alkohol, liegt.

5. Verfahren nach Anspruch 4, **dadurch gekennzeichnet, dass** die verwendete Menge an Phosgen, Diphosgen und/oder Triphosgen derart ist, dass sie zwischen 1,5 und 2,5 Moläquivalenten Phosgen, bezogen auf den Alkohol, liegt.

6. Verfahren nach irgendeinem der Ansprüche 1 bis 5, **dadurch gekennzeichnet, dass** das erhaltene Chlorformiat ein sekundäres oder tertiäres aliphatisches oder cycloaliphatisches Chlorformiat ist.

7. Verfahren nach irgendeinem der Ansprüche 1 bis 6, **dadurch gekennzeichnet, dass** das erhaltene Chlorformiat Menthylchlorformiat ist.

## Claims

1. Process for the synthesis of substituted or unsubstituted aliphatic, cycloaliphatic or araliphatic chloroformates by reaction of the corresponding alcohol with phosgene, diphosgene and/or triphosgene, **characterized in that** the reaction is carried out under a pressure of between 35 × 10³ Pa and 75 × 10³ Pa and at a temperature of between -10°C and +20°C.

2. Process according to Claim 1, **characterized in that** the reaction is carried out in the presence of an inert solvent chosen from the group consisting of chlorinated or nonchlorinated aliphatic hydrocarbons, chlorinated or nonchlorinated aromatic hydrocarbons, esters and ethers.

3. Process according to Claim 1 or 2, **characterized in that** phosgene is used.

4. Process according to any one of Claims 1 to 3, **characterized in that** the amount of phosgene, diphosgene and/or triphosgene used is such that there is between 1 and 10 molar equivalents of phosgene with respect to the alcohol.

5. Process according to Claim 4, **characterized in that** the amount of phosgene, diphosgene and/or triphosgene used is such that there are between 1.5 and 2.5 molar equivalents of phosgene with respect to the alcohol.

6. Process according to any one of Claims 1 to 5, **characterized in that** the chloroformate obtained is a secondary or tertiary aliphatic or cycloaliphatic chloroformate.

7. Process according to any one of Claims 1 to 6, **characterized in that** the chloroformate obtained is menthyl chloroformate.
